# NEUE EUROPÄISCHE PATENTSCHRIFT

(11) **EP 1 006 225 B2**
(45) Veröffentlichungstag und Bekanntmachung des Hinweises auf die Entscheidung über den Einspruch: **18.02.2009**
(45) Hinweis auf die Patenterteilung: 12.11.2003
(21) Anmeldenummer: 99123120.0
(22) Anmeldetag: 18.11.1999
(51) Int. Cl.: D01H 13/32, G01N 33/36

(54) **Verfahren zur Auswertung der Wirkung von Garneigenschaften auf das Aussehen textiler Flächengebilde**
Method for evaluating the effect of yarn characteristics on the looks of textile surfaces
Méthode pour évaluer l'effet des charactéristiques du fil sur l'aspect des surfaces textiles

(30) Priorität: 02.12.1998 DE 19855588
(43) Veröffentlichungstag der Anmeldung: 07.06.2000
(73) Patentinhaber: Oerlikon Textile GmbH & Co. KG, 42897 Remscheid (DE)
(72) Erfinder: Rienas, Gerhard, 52525 Heinsberg (DE)

(56) Entgegenhaltungen:
- EP-A- 0 401 600
- EP-A- 0 531 894
- EP-A- 0 578 975
- EP-A- 0 754 788
- WO-A-97/31262
- WO-A-97/47959
- DE-A- 3 928 417
- DE-A- 19 547 544
- US-A- 5 834 639
- 'Qualitätssicherung der Produktion mit Rotor-Spinnautomaten' USTER NEWS BULLETIN NR: 34 April 1987,
- 'USTER POLYGUARD Q-PACK <On-line Qualitätsüberwachung von Rotorgarnen mit dem USTER POLYGUARD Q-PACK>' USTER NEWS NR. 37 OKT. 1990 Oktober 1990,
- 'USTER POLYGUARD 5/ Anwendungshandbuch', August 1998 Artikel 'Fremdstofferfassung mit USTER POLYGUARD 5-F'

## Beschreibung

Die Erfindung betrifft ein Verfahren zur Auswertung der Wirkung von Garneigenschaften auf das Aussehen textiler Flächengebilde gemäß dem Oberbegriff des Anspruches 1.

Bei der Garnherstellung unterliegen die Kenngrößen eines Garnes periodischen oder zufälligen Schwankungen, die zu unerwünschten Fehlern im Fertigprodukt, zum Beispiel einem aus dem betreffenden Garn hergestellten Gewebe oder Gewirke, führen können, obwohl die Überprüfung des Garnes keine unzulässigen Abweichungen von den Soll-Werten bestimmter Garnparameter, wie dem Durchmesser oder dem Volumen, ergeben hat. Auch wenn die Abweichungen nicht ausreichend zur Aktivierung der Garnreinigung sind beziehungsweise keine Off-Standard-Werte darstellen, können die Abweichungen im fertigen Flächengebilde, zum Beispiel durch ihre Häufung oder Lage, störend wirken, wodurch eine Wertminderung oder Unbrauchbarkeit des gesponnenen Garnes eintreten kann. Die Erfassung und Auswertung der Garnparameter allein ist nicht ausreichend, um einen Eindruck von den Auswirkungen der Garnwerte auf das Aussehen des Fertigproduktes zu vermitteln.

Um Verluste durch die Weiterverarbeitung von unbrauchbarem Garn zu vermeiden oder über dessen mögliche Verwendung vor seiner Weiterverarbeitung zu entscheiden, ist es bekannt, mit dem fertiggesponnenen Garn im Textillabor das Warenbild des Fertigproduktes zu simulieren.

Die EP 0 578 975 A1 geht von einem Stand der Technik aus, bei dem zur Beurteilung der Auswirkung von Garnfehlern auf aus vorliegendem Garn hergestellten Gewebe oder Gewirke zur Simulation des Warenbildes sogenannte Schautafeln verwendet werden. Derartige Schautafeln bestehen aus trapezförmigen oder rechteckigen Kartons oder Blechen und werden mit dem jeweiligen Garn umwickelt. Da dies Voraussagen über eines der wichtigsten Qualitätsmerkmale des Fertigproduktes, nämlich dessen Aussehen und eventuell entstehende Fehlermuster, ermöglicht, werden die Schautafeln als wertvolles Hilfsmittel zur Abschätzung, ob und inwieweit ein bestimmtes Garn für eine bestimmte Ware geeignet ist, bezeichnet. Die Herstellung der Schautafeln durch Umwickeln wird als relativ arbeitsintensiv und nicht mehr zeitgemäß geschildert.

Die EP 0 578 975 A1 offenbart eine Labor-Prüfanlage, mit der die Schautafeln auf elektronischem Wege hergestellt werden können. Die Prüfanlage enthält ein Meßorgan zur Bestimmung von mit dem Volumen und/oder der Oberfläche des Garnes zusammenhängenden Garnparametern, einen Rechner zur Umrechnung der genannten Garnparameter in Grau- oder Farbwerte, Mittel zur Zuordnung der Grau- oder Farbwerte zu Bildpunkten, einen Bildschirm, einen Drucker und Steuermittel zur Wiedergabe der Bildpunkte auf dem Bildschirm oder dem Drucker zwecks Simulation eines aus dem untersuchten Garn hergestellten Gewebes oder Gewirkes. Das Garn wird durch die Meßorgane geführt, die die Masse, die Haarigkeit oder die Struktur fortlaufend messen und in elektrische Signale umwandeln. Das geprüfte Garn wird nach der Messung abgesaugt. In der Auswerte- und Bedienungseinheit erfolgt die Signal- und Datenverarbeitung und die Funktionskontrolle der Prüfanlage. Über eine Tastatur, einen Bildschirm und Steuertasten werden Variable, Meßbedingungen und die gewünschte Darstellung der Resultate eingegeben, und auf dem Bildschirm oder dem Drucker erscheinen Meßablauf und Resultate in numerischer oder grafischer Form. Das Abbild des aus dem untersuchten Garn hergestellten Gewebes oder Gewirkes zeigt dann der Bedienungsperson unmittelbar die Auswirkungen der gefundenen Garnfehler auf das Fertigprodukt und ermöglicht eine Prognose des späteren Warenbildes.

Die Druckschrift "Qualität auf einen Blick! OASYS" der Firma Zweigle offenbart eine Laborprüfeinrichtung mit optischem Sensor, Bildschirm und Drucker, mit der sich aus gemessenen Garnprofilen Wickeltafeln, Gewebe oder Gestricke simulieren und visuell beurteilen lassen. Die Simulation kann sowohl auf dem Bildschirm wie auch auf dem Drucker erfolgen.

Das zu prüfende Garn wird in das Textillabor geschafft und dort häufig in eine Warteschlange eingereiht, so daß sich eine Bewertung tagelang verzögern kann. Mit den bekannten Laborprüfanlagen kann zwar vermieden werden, ein unbrauchbares Fertigprodukt zu erzeugen und dafür beim Weben oder Wirken Kosten und Zeit für die Weiterverarbeitung des Garns aufzuwenden. Das Zwischenprodukt Garn liegt jedoch fertig gesponnen vor und ist bei im Hinblick auf das Fertigprodukt unzureichender Garnqualität nicht mehr oder nur noch bedingt verwendbar. Zum daraus entstehenden Schaden durch Wertminderung oder Unverkäuflichkeit des Garnes kommen möglicherweise zusätzliche Schwierigkeiten bei der Neubeschaffung des Garns und bei der Einhaltung des Liefertermins für das Fertigprodukt hinzu.

Der Erfindung liegt die Aufgabe zugrunde, auftretende Nachteile, die durch Nichteinhalten der gewünschten Garnqualität verursacht werden, zu minimieren.

Diese Aufgabe wird erfindungsgemäß durch ein Verfahren, wie es in Anspruch 1, beschrieben ist, gelöst.

Vorteilhafte Ausgestaltungen der Erfindung sind Gegenstand der Unteransprüche.

Die Erfindung ermöglicht es, zu einem wesentlich früheren Zeitpunkt als bisher Maßnahmen zu ergreifen, wenn das hergestellte Garn nicht den Anforderungen genügt, die für die Erzeugung eines einwandfreien beziehungsweise gewünschten Endproduktes erfüllt sein müssen. Eine Bedienungsperson kann das Bild des simulierten Fächengebildes visuell prüfen und bewerten und Entscheidungen beziehungsweise Maßnahmen treffen. Anhand des Bildes lassen sich das Auftreten von Streifigkeit, Moiré-Mustern oder auch andersfarbigen Fremdfasern deutlich erkennen. Anschließend an die Auswertung des Bildes, das ein aus dem Garn erzeugtes Flächengebilde darstellt, kann sofort auf die Erzeugung des Fadens mit dem Ziel einer Optimierung eingewirkt werden und die Produktionsbedingungen des Garns den Anforderungen des Fertigprodukts angepaßt werden. Das Einwirken auf die Produktion der Spinnvorrichtung bereits während der laufenden Erzeugung des Fadens trägt wirkungsvoll zur Schadensminimierung bei. Die Produktion von unbrauchbarem oder unerwünschtem Garn kann so vermieden werden.

Bevorzugt werden die mindestens einen Garnparameter repräsentierenden Daten abgespeichert und die abgespeicherten Daten dienen nach Erreichen einer bestimmten Datenmenge der Generierung des Bildes. Garnparameter sind zum Beispiel Masse, Durchmesser, Oberflächeneigenschaft, Haarigkeit, Struktur des Garnes sowie Fremdfaserauftreten. Diese Garnparameter repräsentieren für eine visuelle Bewertung besonders relevante Merkmale. Bei der Generierung eines Bildes aus den über einen bestimmten Zeitraum abgespeicherten Daten kann sofort eine größere Datenmenge und damit ein längerer Garnabschnitt zugrunde gelegt werden. Es kann aber auch nach Erreichen eines bestimmten Prüfumfangs, wie zum Beispiel nach dem Durchlauf von 1.000 m Garn, ein Bild generiert werden. Die Verwendung einer bestimmten größeren Menge von abgespeicherten Daten des Garns erhöht die Zuverlässigkeit der Aussage eines daraus generierten Bildes. Die Datenverarbeitung kann auch derart erfolgen, daß nur die die jeweils letzten 1.000 m Garn repräsentierenden Daten abgespeichert bleiben und einer Bildgenerierung dienen. Entsprechend können auch die der Bildgenerierung dienenden Daten nur für eine bestimmte Zeitspanne rückwirkend abgespeichert bleiben.

Durch eine automatisch durchführbare Bildgenerierung erfolgt das Einwirken auf die Garnproduktion umgehend und ohne Beanspruchung der Bedienungsperson. Die Zahl der erforderlichen Monitore kann gering gehalten bzw. auf jeweils einen Monitor beschränkt werden.

Erfindungsgemäß wird der mindestens eine detektierte Garnparameter laufend ausgewertet und bei einer Häufung von Werten, die zwar von einem vorbestimmten Sollwert abweichen, aber als Einzelwert keinen Off-Standard-Wert darstellen, erfolgt die Bildgenerierung. Das Vorliegen eines Bildes wird der Bedienungsperson signalisiert. Eine vorbeugende Überprüfung mittels eines Bildes, die zu einem Zeitpunkt erfolgt, an dem das Ergebnis der Auswertung der Garnparameter auf die erhöhte Wahrscheinlichkeit einer unerwünschten Auswirkung der Garneigenschaften auf das Fertigprodukt hinweist, ist besonders effektiv.

In einer vorzugsweisen Ausbildung des Verfahrens erfolgt eine automatische Auswertung des Bildes durch Vergleich mit mindestens einem Referenzbild. Zum Beispiel werden bestimmte Charakteristika im Bild von der Datenverarbeitungsanlage erkannt und diese Charakteristika bestimmten verstellbaren Parametern der Spinnvorrichtung zugeordnet. Bei unzulässigen Abweichungen vom vorbestimmten Standard wird eine Einwirkung auf diese verstellbaren Parameter der Spinnvorrichtung in Richtung Standard vorzugsweise selbsttätig ausgelöst. Nach einer Bewertung der Charakteristika kann auch eine Klassifizierung des Bildes vorgenommen werden. Die den jeweiligen Anforderungen genügenden Klassen werden vorbestimmt und eine unzulässige Abweichung liegt dann vor, wenn das Bild nicht einer vorbestimmten genügenden Klasse zugeordnet wird. Dadurch wird eine schnelle Auswertung nach reproduzierbaren Kriterien ermöglicht. Das Einwirken auf die Garnproduktion erfolgt umgehend und ohne Beanspruchung der Bedienungsperson. Wird zum Beispiel ein Moiré-Muster erkannt, kann auch selbsttätig eine Reinigung des Rotors eingeleitet werden. Im Rahmen der Erfindung können auch sogenannte Experten- oder Consulting-Systeme zum Einsatz kommen. Zum Beispiel können in einer Datenverarbeitungsanlage Wissensspeicher angelegt werden, die die gegenseitige Zuordnung von Kenngrößen für das Erreichen bestimmter Erzeugniseigenschaften des textilen Fertigproduktes und für die Spinneinrichtung selbst beinhalten. Bei Vorgabe spezifischer Kenngrößen wird durch eine Verknüpfungsschaltung die Zuordnung oder Errechnung der Kenngrößen der Spinneinrichtung vorgenommen. Eine weitergehende Beschreibung eines derartigen Expertensystems kann der Druckschrift EP 0 452 836 B1 entnommen werden.

Alternativ ist die Bildgenerierung abrufbar. Die Bedienungsperson kann sich zum Beispiel in einer Zentrale aufhalten, mit einer zentralen Datenverarbeitungsanlage in Verbindung treten und stichprobenartig das Generieren von Bildern auslösen. Auf diese Weise kann in jedem Falle die Erfahrung und das Urteilsvermögen der Bedienungsperson in die visuelle Beurteilung einfließen. In einer weiteren Ausbildung des Verfahrens erfolgt bei von der Datenverarbeitungsanlage erkannten unzulässigen Abweichungen vom vorbestimmten Standard beziehungsweise bestimmten Charakteristika eine abrufbare Bildgenerierung beziehungsweise es wird ein entsprechendes Signal für die Bedienungsperson erzeugt. Die Datenverarbeitungsanlage kann hierbei einen Vorschlag für auf die Garnproduktion einwirkende Maßnahmen erstellen. Die Bedienungsperson kann entweder diesem Vorschlag folgen oder nach eigenem Ermessen tätig werden. Die Aufmerksamkeit der Bedienungsperson wird nur bei einer erkannten Abweichung beziehungsweise einem erkannten Mangel beansprucht. Gleichzeitig wird weniger Rechnerkapazität benötigt.

Das Einwirken über verstellbare Parameter der Spinnvorrichtung in Abhängigkeit vom Ergebnis der Abweichung, wobei das Einwirken auch selbsttätig, zum Beispiel durch die Datenverarbeitungsanlage gesteuert, erfolgen kann, beeinflußt den Spinnprozess umgehend, ist reproduzierbar und eignet sich besonders gut für eine Automatisierung. Der erforderliche Umfang der Veränderung von verstellbaren Parametern der Spinnvorrichtung wird jeweils empirisch ermittelt und abgespeichert. Die ermittelten Werte der verstellbaren Parameter stehen für spätere Spinnprozesse zum Beispiel als Matrix zur Verfügung.

Die Einwirkung auf die Erzeugung des Fadens durch ein Einwirken auf die Bewertung der Ergebnisse der Sensoreinrichtung kann zum Beispiel derart erfolgen, daß die Einstellung des Reinigers beziehungsweise der Ausreinigungsgrad den jeweiligen Anforderungen an das Garn angepaßt werden. Das heißt, die Anzahl der durch den Reiniger bewirkten Fadenschnitte kann gegebenenfalls auch gesenkt und somit die Produktivität erhöht werden.

Das Einwirken auf die Erzeugung des Fadens über die Ausbildung sowie den Einsatz oder Austausch mindestens eines Spinnmittels führt zum Beispiel zur Auswahl der geeignetsten Spinnmittel aus vorhandenen Rotortypen, Abzugsdüsen oder Auflösewalzen. Auch bei der Neuentwicklung derartiger Spinnmittel können die Auswirkungen von Ausbildungsvarianten auf das zu erzeugende Flächengebilde bewertet und die für den jeweiligen Spinnprozeß geeignetsten Varianten ausgewählt werden. Bei Abweichungen, die zum Beispiel durch Verschleiß oder Defekt der Spinnmittel verursacht werden, kann der Zusammenhang der Charakteristik dieser Abweichung mit Verschleißerscheinungen oder Defekt der jeweiligen Spinnmittel in der Datenverarbeitungsanlage abgespeichert werden und bei Wiederauftreten einer derartigen Abweichung zielgerichtet eine bedarfsgesteuerte Wartung ausgelöst werden. Dadurch lassen sich die Wartungsintervalle vergrößern, ohne daß es zu unzulässigen Qualitätseinbußen des Garnes kommt.

Anspinner können im Fertigprodukt erkennbar sein. Daher werden zum Beispiel nach einem Partiewechsel bei neuem Material mehrere Anspinner erzeugt und deren Auswirkungen auf das Aussehen des Fertigproduktes einer Bewertung unterzogen. Wirken die Anspinner im Fertigprodukt störend, kann zur Verbesserung das Erzeugen des Anspinners beziehungsweise das für das Anspinnen eingesetzte Aggregat und dessen Funktion optimiert und die Beeinträchtigung im Aussehen des Fertigproduktes beseitigt werden. Auch hier kann ein Experten- oder Consulting-System zum Einsatz kommen. Zum Beispiel wird die Auswirkung von unterschiedlich profilierten Dick- und Dünnstellen und deren Lage zum Anspinner auf das Aussehen des Fertigproduktes beurteilt und das Ergebnis der Beurteilung dient als Grundlage für die Änderung der Kriterien, Parameter und Sollwerte künftiger automatischer Anspinnvorgänge. Die Sollwerteinsteller eines Anspinnrechners können beispielsweise aufgrund des Bewertungsergebnisses entweder manuell oder über eine Schnittstelle automatisch neu eingestellt werden. Weitere Einzelheiten eines solchen Systems beschreibt die DE 40 30 100 A1.

Das dargestellte Flächengebilde ist zum Beispiel eine Garntafel. Mit Hilfe einer Garntafel kann auf einfache und schnelle Weise zum Beispiel auf das zu erwartende Auftreten von Querstreifen oder Moiré-Mustern im Fertigprodukt rückgeschlossen werden. Vorzugsweise ist das dargestellte Flächengebilde ein aus dem geprüften Garn zu fertigendes Flächengebilde wie zum Beispiel ein Gewebe oder Gewirke. Eine derartige Darstellung zeigt besonders effektiv und wirklichkeitsnah die Auswirkung von detektierten Garneigenschaften auf das fertige Endprodukt.

Insbesondere dient zur Erfassung von Garnparametern ein optisches Meßverfahren. Auch der Einsatz eines kombinierten optisch-kapazitiven Meßverfahrens bietet Vorteile, weil damit auch zusätzliche Aussagen zum Beispiel zur Dichte des überprüften Fadens möglich sind. Auf diese Weise können besonders geeignete Garnparameter zuverlässig und ohne Schwierigkeiten gemessen, erfaßt und verglichen werden.

Zur Auswertung des Flächengebildes dient bevorzugt die optisch dargestellte Häufigkeit und Verteilung von Fremdfasern sowie von Ungleichmäßigkeiten, wie Dick- und Dünnstellen oder Nissen. Auf diese Weise werden besonders häufige Abweichungen, die das gleichmäßige Aussehen des Flächengebildes stören, erfaßt. Die ermittelten Abweichungen können aber auch klassifiziert, einer Matrix zugeordnet und mindestens eine als zulässig bewertete Klasse vorgegeben werden. Bewegt sich das Meßergebnis nicht innerhalb einer vorgegebenen zulässigen Klasse, wird dies als Abweichung erkannt.

Es kann zweckmäßig sein, nach einem Partiewechsel zunächst an einer einzelnen oder an wenigen Spinnstellen mit der Garnherstellung für einen bestimmten Verwendungszweck zu starten, nach einer bestimmten Garnlänge ein Bild zu generieren und, falls erforderlich, Maßnahmen beziehungsweise Einstellungen zum Einhalten des benötigten oder gewünschten Standards durchzuführen und dann mit diesen Einstellungen die restlichen Spinnstellen zu starten.

In bestimmten Fällen wird aus Kostengründen billigeres Fasermaterial eingesetzt beziehungsweise beigemischt. Über eine Bildgenerierung mit anschließender Auswertung kann festgestellt werden, ob das Aussehen des Fertigproduktes den Anforderungen noch genügen wird, wenn ein Garn aus derartigem Material oder mit derartigen Beimischungen verarbeitet wird.

Die Verbindung mindestens einer in einer Spinnvorrichtung angeordneten Sensoreinrichtung zur Detektierung mindestens eines Garnparameters während der laufenden Erzeugung des Fadens mit mindestens einem Mittel zur Auswertung von Garnparametern sowie zur Bildgenerierung erlaubt einerseits, bereits in der Spinnvorrichtung vorhandene Sensoreinrichtungen zu nutzen, andererseits ist die Detektierung von Garnparametern bereits zu einem sehr frühen Zeitpunkt möglich. Bevorzugt ist die Sensoreinrichtung als optisch-kapazitiver Reiniger ausgebildet und ist so besonders gut zur Erfassung relevanter Garnparameter geeignet.

Das Mittel zur Bildgenerierung ist vorzugsweise ein Monitor, alternativ oder zusätzlich auch ein Drucker. Diese Ausbildungsformen liefern eine besonders schnelle und reproduzierbare Darstellung der simulierten Flächengebilde.

In Weiterbildung weist die Vorrichtung mindestens ein Mittel zur selbsttätigen Beeinflussung der Erzeugung des Fadens auf. Derartige Mittel können zum Beispiel regelbare Antriebsaggregate für die Einzugswalze, die Auflösewalze, den Rotor oder die Abzugswalzen sein. Damit kann zum Beispiel auf den Durchmesser oder die Masse des Fadens eingewirkt werden. Auf diese Weise kann umgehend und wirkungsvoll, ohne Zeit und Aufmerksamkeit der Bedienungsperson in Anspruch zu nehmen, auf verstellbare Parameter der Spinnvorrichtung in Abhängigkeit von der Abweichung eingewirkt werden.

Das Mittel zur Auswertung von Garnparametern sowie zur Bildgenerierung ist bevorzugt jeweils mit einer Anzahl von Spinnvorrichtungen verbunden. Dadurch kann die Anzahl von Mitteln zur Auswertung und Bildgenerierung, insbesondere die Anzahl von benötigten Bildschirmen oder Druckern, wesentlich gesenkt werden. Das Mittel kann dabei nach Art des Timesharing-Verfahrens für die einzelne Spinnvorrichtung eingesetzt werden. Es können auch abgespeicherte Daten der jeweiligen Spinnvorrichtung nacheinander zur Bildgenerierung auf demselben Bildschirm oder demselben Drucker dienen. Durch die Verbindung der Spinnvorrichtung mit mindestens einem Mittel, das die Einzelabfrage von Spinnvorrichtungen beziehungsweise von Spinnstellen ermöglicht, können Spinnvorrichtungen beziehungsweise Spinnstellen gezielt und mittels gemeinsamer Nutzung eines Monitors oder Druckers überwacht werden. Es ist nur ein Monitor beziehungsweise ein Drucker für eine Spinnmaschine mit einer Vielzahl von Spinnstellen notwendig und die erforderliche Rechenkapazität kann gering gehalten werden.

In einer weiteren Ausführungsform werden die Daten einer zentralen Informations- oder Datenverarbeitungsanlage zugeleitet, dort abgespeichert sowie verarbeitet, weitergegeben oder von dort abgespeicherte sowie verarbeitete Daten abgerufen. Auf diese Weise ist ein Abgleich mit anderen Spinnstellen besonders einfach und effektiv durchführbar. Die erforderliche Rechenkapazität in Spinnereien mit einer Mehrzahl von Spinnmaschinen kann minimiert und anstelle einer Mehrzahl von Monitoren braucht nur ein einziger Monitor eingesetzt zu werden.

Die Erfindung erlaubt ein frühes Erkennen von Einflüssen der Garnparameter auf das Aussehen des Fertigproduktes und ein Einwirken auf die Herstellung des Garns zum Einhalten des gewünschten beziehungsweise erforderlichen Standards. Verluste an Geld und Zeit durch unbrauchbares oder nur eingeschränkt verwendbares Garn können so minimiert oder ganz vermieden werden.

Weitere Einzelheiten der Erfindung sind den Darstellungen der Figuren entnehmbar.

Es zeigt:
- Fig. 1:: eine schematische Darstellung ein Open-End-Spinnaggregat in Seitenansicht sowie Mittel zur Datenverarbeitung und Bildgenerierung,
- Fig. 2:: ein simuliertes Flächengebilde mit streifigem Warenbild.

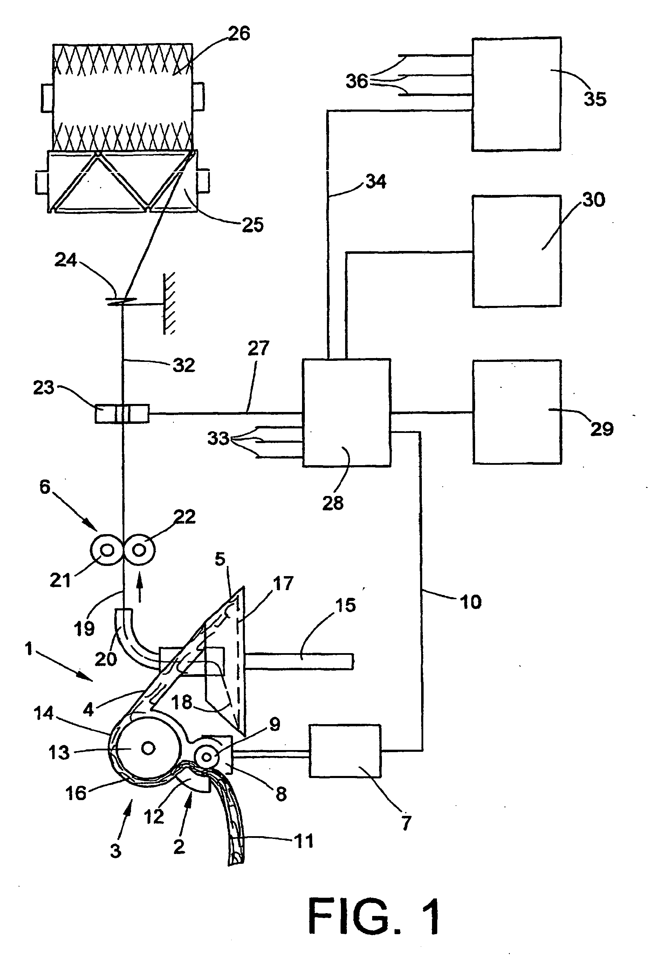

Als Spinnvorrichtung wird in Figur 1 ein Open-End-Spinnaggregat 1 einer Open-End-Spinnmaschine dargestellt. Eine Open-End-Spinnmaschine besitzt eine Mehrzahl von Open-End-Spinnaggregaten 1 mit jeweils einer Faserbandeinzugsvorrichtung 2, einer Auflösevorrichtung 3, einem Faserkanal 4, einem Rotor 5 und einer Abzugsvorrichtung 6.

Die Faserbandeinzugsvorrichtung 2 weist eine durch einen stufenlos regelbaren Elektromotor 7 über eine Getriebeeinrichtung 8 angetriebene Einzugswalze 9 auf. Die Regelung des Elektromotors 7 erfolgt über die Steuerleitung 10. Das Faserband 11 wird zwischen Einzugsmulde 12 und Einzugswalze 9 hindurch zu der Auflösevorrichtung 3 transportiert. Die Auflösevorrichtung 3 besitzt eine gezahnte Auflösewalze 13, die in einem Gehäuse 14 rotiert. Der Faserkanal 4 reicht vom Gehäuse 10 der Auflösevorrichtung 3 bis zum Rotor 5. Der Rotor 5 ist mit der Rotorwelle 15 verbunden, die in bekannter, hier aus Vereinfachungsgründen nicht dargestellter Weise angetrieben und gehaltert ist. Die durch die Auflösevorrichtung 3 vereinzelten Fasern 16 gelangen durch den Faserkanal 4 in die Fasersammelrille 17 des rotierenden Rotors 5. In der Fasersammelrille 17 legen sich die dort befindlichen Fasern 16 an das Fadenende 18 an und werden von dem Rotor 5 in eine Drehbewegung versetzt. Der zum Fadenende 18 gehörende Faden 19 wird durch ein Fadenabzugsrohr 20 in Pfeilrichtung mittels der Abzugsvorrichtung 6 abgezogen. Die Abzugsvorrichtung 6 weist zwei als Walzenpaar angeordnete Walzen 21, 22 auf. Nach der Abzugsvorrichtung 6 durchläuft der gesponnene Faden eine als Reiniger ausgebildete Sensoreinrichtung 23 sowie einen Fadenführer 24 und wird über eine Andrückwalze 25 der als Kreuzspule ausgebildeten Auflaufspule 26 zugeführt. Die Sensoreinrichtung 23 kann alternativ auch zwischen dem Fadenabzugsrohr 20 und den Walzen 21, 22 der Abzugsvorrichtung 6 angeordnet sein. Über die Leitung 27 ist die Sensoreinrichtung 23 mit einer Datenverarbeitungsanlage 28 verbunden. Die von der Sensoreinrichtung detektierten Garnparameter werden in Daten umgewandelt und gespeichert. Die Daten dienen der Generierung eines Bildes, das auf dem Bildschirm des Monitors 29 oder mittels des Druckers 30 dargestellt wird. Es ist möglich, ein Bild erst dann zu generieren, wenn die vorliegenden Daten eine bestimmte Garnmenge repräsentieren, zum Beispiel, wenn 1.000 m Garn 32 die Sensoreinrichtung 23 durchlaufen haben, oder eine vorbestimmte Zeit verstrichen ist. Über die Leitungen 33 sind weitere aus Vereinfachungsgründen nicht dargestellte Open-End-Spinnaggregate beziehungsweise Spinnstellen mit der Datenverarbeitungsanlage 28 verbunden. Die Datenverarbeitungsanlage 28 ist über die Leitung 34 an eine zentrale Datenverarbeitungsanlage 35 angeschlossen, die ihrerseits durch die Leitungen 36 mit weiteren ebenfalls zur Vereinfachung nicht dargestellten Datenverarbeitungsanlagen beziehungsweise Spinnstellen verbunden ist.

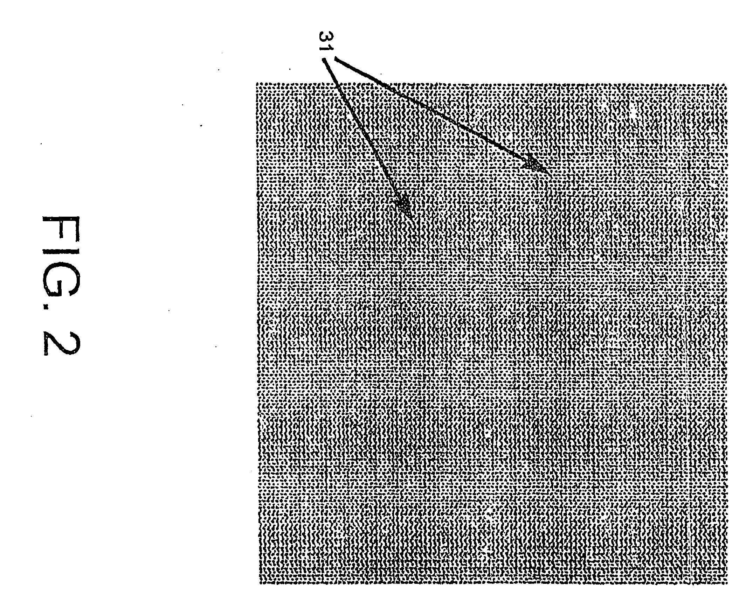

Figur 2 zeigt die aus detektierten Garnparametern eines Garnes 32 generierte Simulation eines Gestrickes, das deutlich erkennbare Streifen 31 aufweist. Die Bedienungsperson entscheidet anhand derartiger Bilder, ob und welche Maßnahmen ergriffen werden, um auf die Erzeugung des Fadens 19 einzuwirken. Die Maßnahmen bestehen zum Beispiel in einer Veränderung von verstellbaren Parametern des Spinnprozesses oder in einem Austausch von einem oder mehreren Spinnmitteln 5,13 oder in Veränderungen des in Form eines Faserbandes 11 zugeführten Fasermaterials.

Die Datenverarbeitungsanlage 28 kann die Auswertung des generierten Bildes auch selbsttätig durch Vergleich des generierten Bildes mit einem Referenzbild durchführen. Das Referenzbild kann durch Fotografieren eines realen Gewebes oder Gewirkes und anschließende Digitalisierung des Fotos erstellt werden. Als Referenzbild können aber auch Bilder simulierter Flächengebilde dienen, die den Anforderungen an das Fertigprodukt genügen. Wenn beim Vergleich des generierten Bildes mit dem Referenzbild eine Abweichung auftritt, die bestimmte Toleranzbereiche überschreitet, erzeugt die Datenverarbeitungsanlage 28 ein optisches oder akustisches Signal, um die Aufmerksamkeit der Bedienungsperson zu erregen. Zusätzlich erstellt die Datenverarbeitungsanlage 28 dabei Vorschläge, welche Maßnahmen zweckmäßig ergriffen werden sollten, um auf die Erzeugung des Fadens 19 einzuwirken. Die Bedienungsperson entscheidet dann, ob und welche Maßnahmen durchgeführt werden. Die Datenverarbeitungsanlage 28 kann aber auch so programmiert sein, daß sie beim Erkennen von als unzulässig eingestuften Abweichungen eine geeignete Veränderung verstellbarer Parameter der Spinnvorrichtung im Sinne einer Optimierung selbsttätig vornimmt. Dies kann beispielsweise in einer Erhöhung oder Verringerung der Faserzuführung bestehen, die durch Regelung der Drehzahl des Elektromotors 7 erfolgt und über die Steuerleitung 10 durch die Datenverarbeitungsanlage 28 selbsttätig vorgenommen wird. Weitere nicht dargestellte Möglichkeiten, eine Veränderung verstellbarer Parameter der Spinnvorrichtung vorzunehmen, bestehen in Mitteln zur Einwirkung auf die Drehzahl des Rotors 5, der Walzen 21 und 22 der Abzugsvorrichtung 6 oder der Auflösewalze 13 der Auflösevorrichtung 3 sein. Die Veränderung kann zum Beispiel mittels regelbarem Elektromotor oder regelbarem Getriebe vorgenommen werden. Das Einwirken kann jeweils auf eine einzelne oder eine Vielzahl von Spinnstellen erfolgen.

## Patentansprüche

1. Verfahren zur Auswertung der Wirkung von Garneigenschaften auf das Aussehen textiler Flächengebilde, bei dem eine Bildgenerierung aus Daten erfolgt, die mindestens einen Parameter eines Garns (32) repräsentieren und das Bild ein aus dem Garn (32) erzeugtes Flächengebilde darstellt,
wobei der mindestens eine Garnparameter während der laufenden Erzeugung des Fadens (19) detektiert, in Daten umgewandelt und einer Datenverarbeitungsanlage (28) zugeführt wird, wobei von der Datenverarbeitungsanlage (28) ein Bild generiert wird und das Bild einer Auswertung zugeführt wird und wobei abhängig vom Ergebnis der Auswertung des Bildes auf die Erzeugung des Fadens (19) eingewirkt wird, wobei die Bildgenerierung automatisch durchführbar ist
**dadurch gekennzeichnet,**
**dass** der mindestens eine detektierte Garnparameter laufend ausgewertet wird und bei einer Häufung von Werten, die zwar von einem vorbestimmten Sollwert abweichen, aber als Einzelwert keinen Off-Standard-Wert darstellen, die Bildgenerierung erfolgt.

2. Verfahren nach Anspruch 1, **dadurch gekennzeichnet, dass** eine automatische Auswertung des Bildes durch Vergleich mit mindestens einem Referenzbild erfolgt.

3. Verfahren nach Anspruch 1 oder 2, **dadurch gekennzeichnet, dass** die Bildgenerierung abrufbar ist und das Vorliegen eines abrufbaren Bildes der Bedienungsperson signalisiert wird.

4. Verfahren nach einem der vorhergehenden Ansprüche, **dadurch gekennzeichnet, dass** das Einwirken auf die Erzeugung des Fadens (19) **dadurch** erfolgt, dass auf verstellbare Parameter der Spinnvorrichtung eingewirkt wird.

5. Verfahren nach einem der vorhergehenden Ansprüche, **dadurch gekennzeichnet, dass** das Einwirken auf die Erzeugung des Fadens (19) **dadurch** erfolgt, dass auf die Bewertung der Ergebnisse der Sensoreinrichtung (23) eingewirkt wird.

6. Verfahren nach einem der vorhergehenden Ansprüche, **dadurch gekennzeichnet, dass** das Einwirken auf die Erzeugung des Fadens (19) über die Ausbildung oder den Austausch mindestens eines Spinnmittels (5, 13) erfolgt.

7. Verfahren nach einem der vorhergehenden Ansprüche, **dadurch gekennzeichnet, dass** auf die Verbesserung des Anspinners eingewirkt wird.

8. Verfahren nach einem der vorhergehenden Ansprüche, **dadurch gekennzeichnet, dass** das dargestellte Flächengebilde eine Garntafel ist.

9. Verfahren nach einem der Ansprüche 1 bis 7, **dadurch gekennzeichnet, dass** das dargestellte Flächengebilde ein Gewebe oder Gewirke ist.

10. Verfahren nach einem der vorhergehenden Ansprüche, **dadurch gekennzeichnet, dass** zur Erfassung von Garnparametern ein optisches Messverfahren dient.

11. Verfahren nach Anspruch 10, **dadurch gekennzeichnet, dass** zur Erfassung von Garnparametern ein kombiniertes optischkapazitives Messverfahren dient.

12. Verfahren nach einem der vorhergehenden Ansprüche, **dadurch gekennzeichnet, dass** zur Auswertung des Flächengebildes die optisch dargestellte Häufigkeit und Verseilung von Fremdfasern sowie von Ungleichmäßigkeiten, wie Dick- und Dünnstellen oder Nissen, dient.

## Claims

1. Process for evaluating the effect of yarn properties on the appearance of textile fabrics, in which an image generation takes place from data which represent at least one parameter of a yarn (32) and the image represents a fabric produced from the yarn (32), wherein the at least one yarn parameter is detected during the routine production of the thread (19), is converted into data and is supplied to a data processing system (28), wherein an image is generated by the data processing system (28) and the image is supplied for evaluation and wherein the production of the thread (19) is influenced in dependence on the result of the evaluation of the image, wherein the image generation can be performed automatically,
**characterised in that**
the at least one detected yarn parameter is routinely evaluated and the image generation takes place in the event of an accumulation of values, which deviate from a predetermined set value but as individual values do not represent an off-standard value.

2. Process according to claim 1, **characterised in that** an automatic evaluation of the image takes place by comparison with at least one reference image.

3. Process according to claim 1 or 2, **characterised in that** the image generation is retrievable and the presence of a retrievable image is signalled to the operator.

4. Process according to one of the preceding claims, **characterised in that** the production of the thread (19) is influenced by the fact that adjustable parameters of the spinning device are influenced.

5. Process according to one of the preceding claims, **characterised in that** the production of the thread (19) is influenced by the fact that the evaluation of the results of the sensor device (23) is influenced.

6. Process according to one of the preceding claims, **characterised in that** the production of the thread (19) is influenced by the configuration or the exchange of at least one spinning means (5, 13).

7. Process according to one of the preceding claims, **characterised in that** the improvement of the piecer is influenced.

8. Process according to one of the preceding claims, **characterised in that** the represented fabric is a yarn panel.

9. Process according to one of claims 1 to 7, **characterised in that** the represented fabric is a woven fabric or a knitted fabric.

10. Process according to one of the preceding claims, **characterised in that** an optical measurement process is used for recording yarn parameters.

11. Process according to claim 10, **characterised in that** a combined optical-capacitive measurement process is used for recording yarn parameters.

12. Process according to one of the preceding claims, **characterised in that** the optically represented frequency and distribution of foreign fibres as well as of irregularities, such as nibs and thin places or neps, serves for the evaluation of the fabric.

## Revendications

1. Procédé pour évaluer l'effet des caractéristiques d'un fil sur l'aspect de surfaces textiles, selon lequel on crée une image à partir de données qui représentent au moins un paramètre d'un fil (32), et l'image représente une surface produite à partir du fil (32), ledit au moins un paramètre du fil étant détecté au cours de la production continue du fil (19), converti en données et transmis à une unité de traitement de données ou un ordinateur (28), l'unité ou l'ordinateur (28) créant une image qui est soumise à une analyse ou évaluation, la production du fil (19) étant influencé en fonction du résultat de l'évaluation de l'image, la création d'image pouvant se faire automatiquement, procédé **caractérisé en ce qu'**au moins l'unique paramètre du fil qui est détecté est analysé ou évalué en continu, et que, lors d'une accumulation de valeurs qui, certes, diffèrent d'une valeur de consigne prédéfinie, mais ne constituent pas isolément une valeur non standard, l'image est créée.

2. Procédé selon la revendication 1, **caractérisé en ce que** l'image est analysée automatiquement en la comparant à au moins une image de référence.

3. Procédé selon la revendication 1 ou 2, **caractérisé en ce que** la création d'image peut être demandée, et la présence d'une image interrogeable est signalée à l'opérateur.

4. Procédé selon l'une des revendications précédentes, **caractérisé en ce que** l'action sur la production du fil (19) consiste à influer sur des paramètres réglables du métier à filer.

5. Procédé selon l'une des revendications précédentes, **caractérisé en ce que** l'action sur la production du fil (19) consiste à influer sur l'appréciation des résultats relevés par le capteur (23).

6. Procédé selon l'une des revendications précédentes, **caractérisé en ce que** l'action sur la production du fil (19) s'effectue par le biais de la réalisation ou du remplacement d'au moins un moyen de filage (5, 13).

7. Procédé selon l'une des revendications précédentes, **caractérisé en ce que** l'on agit pour améliorer le fileur.

8. Procédé selon l'une des revendications précédentes, **caractérisé en ce que** la surface représentée est une table de conversion des fils.

9. Procédé selon l'une des revendications 1 à 7, **caractérisé en ce que** la surface représentée est un tissu ou un tricot.

10. Procédé selon l'une des revendications précédentes, **caractérisé en ce que**, pour relever des paramètres du fil, on utilise un procédé de mesure optique.

11. Procédé selon la revendication 10, **caractérisé en ce que**, pour relever des paramètres du fil, on utilise un procédé de mesure combinant des mesures optique et capacitive.

12. Procédé selon l'une des revendications précédentes, **caractérisé en ce que**, pour analyser la surface, on examine la fréquence et la répartition représentées par voie optique de fibres étrangères et d'irrégularités telles que des segments épais et minces ou des boutons.
